# FASCICULE DE BREVET EUROPEEN

(11) **EP 0 492 196 B1**
(45) Date de publication et mention de la délivrance du brevet: **22.02.1995**
(21) Numéro de dépôt: 91120805.6
(22) Date de dépôt: 04.12.1991
(51) Int. Cl.: A61M 25/01

(54) **Support de guidage pour cathéter**
Führungstütze für Katheter
Guidance support for catheter

(30) Priorité: 28.12.1990 FR 9016579
(43) Date de publication de la demande: 01.07.1992
(73) Titulaire: Nivarox-FAR S.A., CH-2400 Le Locle (CH)
(72) Inventeur: Grenouillet, Guy, F-25130 Villers-Le-Lac (FR)
(74) Mandataire: de Raemy, Jacques

(56) Documents cités:
- EP-A- 0 200 919
- WO-A-89/10088
- WO-A-90/01892
- CH-A- 674 943
- US-A- 4 721 117

## Description

L'invention concerne les supports de guidage en général et plus particulièrement un support de guidage destiné à être introduit et guidé dans des vaisseaux sanguins de faibles diamètres pour faciliter l'introduction d'un cathéter dans ceux-ci.

Ce type de support de guidage pour cathéter trouve de nombreuses applications notamment dans le cadre de la chirurgie cardiovasculaire comme, par exemple, lors d'une angioplastie coronarienne, pendant laquelle on introduit un cathéter dans une région rétrécie de l'artère coronaire du patient et on gonfle le cathéter pour dilater le passage de l'artère.

On connaît déjà du document US 4 545 390 un support de guidage pour cathéter, ce dernier présentant un faible diamètre (≦ 0,5 mm) et étant destiné à être introduit par voie endoveineuse dans le corps humain.

Selon ce document, le support de guidage comprend un fil en acier inoxydable comportant dans une région distale une zone effilée sur laquelle est enfilée un ressort hélicoïdal présentant un diamètre sensiblement égal au diamètre du fil principal. L'extrémité proximale du ressort est fixée sur le fil à l'aide d'une brasure dans la région du fil où celui-ci commence à s'effiler, et l'extrémité distale du ressort est également fixée par brasure à l'extrémité distale du fil.

Un tel support de guidage, fonctionnant généralement d'une façon satisfaisante, présente toutefois des inconvénients.

En effet, lors de la fixation par brasage du ressort sur le fil, on chauffe localement ce dernier à des températures (≅ 500 °C) avoisinant les températures de début de transformation cristallographique des aciers inoxydables et qui correspondent au recuit de ces derniers, et auxquelles les propriétés mécaniques de ces aciers commencent à se détériorer de façon très sensible. On comprend donc aisément que ce recuit, apparaissant lors de l'opération de brasage, peut conduire à une rupture prématurée du fil lors de son utilisation, une telle rupture pouvant causer de graves complications si elle se produit dans la veine d'un patient.

Par ailleurs, les supports de guidage tels que ceux décrits dans le document mentionné présentent une surépaisseur à l'endroit de la soudure qui risque de gêner, voire de bloquer le cathéter lorsque celui-ci glisse sur le support.

De plus, quand le fil, généralement revêtu d'une couche d'un matériau à faible coefficient de frottement tel que le TEFLON ^{R} , est associé à un ressort hélicoïdal revêtu d'une couche d'or pour des raisons de biocompatibilité, le brasage du ressort sur le fil provoque une détérioration à la fois de la couche d'or et de la couche de matériau à faible coefficient de frottement nuisible à la biocompatibilité du support de guidage.

L'invention a donc pour but principal de remédier aux inconvénients de l'art antérieur décrit ci-dessus en fournissant un support de guidage pour cathéter présentant une meilleure fiabilité.

A cet effet, l'invention a pour objet un support de guidage pour cathéter comprenant un fil ayant une partie proximale de section sensiblement constante et une partie distale effilée, et une gaine de protection souple s'étendant au moins sur la longueur de la partie distale du fil.

Conformément à l'invention, le fil est rendu solidaire de la gaine uniquement par l'extrémité libre de sa partie distale au moyen d'une première soudure formant un bout arrondi et le support comprend en outre un tube s'étendant sur toute la partie du fil non recouverte par la gaine, une extrémité du tube venant simplement en butée contre la partie proximale de la gaine tandis que l'extrémité opposée du tube est fixée à la partie proximale du fil au moyen d'une seconde soudure.

Grâce à ces caractéristiques, on élimine le soudage de la gaine de protection sur une partie utile du fil. Ainsi, les propriétés mécaniques du fil dans ses parties mécaniquement sollicitées, notamment en flexion, lors de la mise en oeuvre du support dans le corps d'un patient ne risquent pas d'être altérées, par exemple par un recuit de la matière à l'endroit de la soudure lors du soudage de la gaine sur le fil, de sorte que la fiabilité générale du support est augmentée.

Selon un mode de réalisation avantageux de l'invention, le fil comprend en outre, à l'extrémité libre de sa partie distale, un tronçon de renfort présentant une section sensiblement égale à la section proximale du fil.

Un tel tronçon de renfort rigidifie l'extrémité du support, et facilite l'introduction de ce dernier dans les veines du patient, notamment dans leurs passages sinueux.

D'autres caractéristiques et avantages de l'invention apparaîtront à la lecture de la description qui suit de modes de réalisation de l'invention, donnés à titre illustratif et non limitatif, en liaison avec les dessins ci-joints parmi lesquels :
- La figure 1 est une coupe fragmentée d'un mode de réalisation du support de guidage selon l'invention; et
- La figure 2 est une coupe de la partie distale d'un autre mode de réalisation du support de guidage selon l'invention.

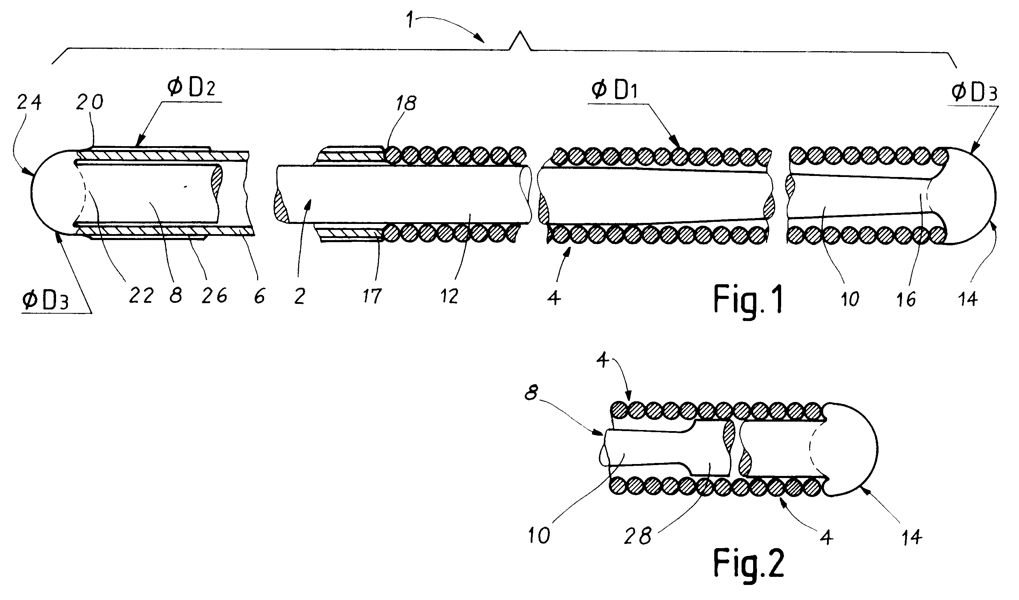

En se référant à la figure 1, on voit un support de guidage pour cathéter selon l'invention désigné par la référence générale 1. Un tel support de guidage est couramment utilisé en médecine lors de la mise en oeuvre de procédés d'angioplastie, notamment pour faciliter l'introduction et le positionnement d'un cathéter tel qu'un cathéter d'exploration, de traitement ou analogue dans le système cardiovasculaire d'un patient. Pour la mise en place du cathéter, il est donc nécessaire d'introduire, dans un premier temps, le support de guidage jusqu'à la zone à traiter du système cardiovasculaire puis, dans un second temps, de faire glisser le cathéter désiré sur le support jusqu'à la zone à traiter.

Comme cela apparaît clairement à la figure 1, le support de guidage pour cathéter comprend un fil 2 formant l'âme du support sur lequel sont enfilés et rendus solidaires du fil une gaine de protection 4 et un tube 6.

Le fil 2, réalisé de préférence en acier inoxydable pour des raisons de biocompatibilité comprend une partie proximale 8 de section constante et une partie distale 10 effilée.

La partie proximale 8 présente une forme générale cylindrique et la partie distale 10 présente une forme générale tronconique.

Typiquement, le diamètre de la partie 8 à section constante du fil est de l'ordre de 0,5 mm et le diamètre de l'extrémité de la partie distale du fil est de l'ordre de 0,1 mm.

La gaine de protection 4 est formée par un ressort hélicoïdal à spires jointives. Cette gaine s'étend sur la partie distale 10 du fil et légèrement au-delà sur une portion 12 cylindrique du fil sur laquelle elle repose. On notera à ce propos que le diamètre intérieur de la gaine est sensiblement ajusté sur le diamètre de la partie proximale du fil.

Conformément à l'invention, la gaine 4 est fixée au fil 2 par une seule et unique soudure 14 réalisée à l'extrémité libre 16 de la partie distale 10 du fil.

La soudure 14 forme avantageusement un bout arrondi de sorte qu'elle minimise les risques de blessures des parois internes du système cardiovasculaire lors de l'introduction du support.

Le tube 6, enfilé sur toute la partie proximale 8 du fil qui n'est pas recouverte par la gaine 4, vient simplement en butée par une extrémité 17 contre la partie proximale 18 de la gaine 4 et est fixé par son extrémité 20 opposée à l'extrémité 22 de la partie proximale 8 du fil au moyen d'une soudure 24, présentant également un bout arrondi.

De préférence, et comme cela est représenté à la figure 1, le diamètre D₁ extérieur de la gaine 4 et celui D₂ du tube 6 sont sensiblement égaux. Typiquement, le diamètre de ces deux éléments est inférieur ou égal à 0,8 mm selon les applications spécifiques du support.

En outre, on remarque que le diamètre D₃ des bouts arrondis est aussi sensiblement égal au diamètre du tube 6 et à celui de la gaine 4. Ainsi, de par la forme arrondie et la dimension du bout, le risque de lésion des parois internes des veines lors de l'introduction du support est fortement diminué et l'introduction du support est grandement facilitée.

On peut voir également que dans le mode de réalisation du support représenté, le tube 6 est revêtu dans son intégralité d'une couche 26 d'un matériau à faible coefficient de frottement tel que du TEFLON ^{R} . Cette couche 26 facilite d'une part l'introduction du support dans le système cardiovasculaire du patient et d'autre part et surtout le glissement du cathéter sur le support quand ce dernier est installé dans le corps du patient.

On comprend aisément qu'avec la structure d'un support selon l'invention, le tube 6 peut être préalablement recouvert de la couche 26 sans que celle-ci soit détériorée dans des zones actives du support au moment où le tube ou la gaine est soudé sur le fil puisque, selon l'invention, on a éliminé toutes les soudures de la partie active du fil pour les déplacer dans des zones neutres, c'est-à-dire dans des zones non fonctionnelles.

La gaine 4 est réalisée dans l'exemple représenté en un métal biocompatible présentant de préférence des propriétés radioabiles pour rendre visible par fluoroscopie l'extrémité du support et ainsi permettre un guidage et un positionnement précis du support dans le système cardiovasculaire. A titre d'exemple, un alliage de platine et d'iridium, comprenant 70 à 80 % de platine et 30 à 20 % d'iridium est adapté pour la réalisation de la gaine 4.

Il est bien entendu que dans le cas où l'alliage radioabile formant la gaine n'est pas biocompatible, comme par exemple le tungstène, on peut recouvrir la gaine d'un métal de placage lui-même biocompatible, tel que l'or. Là encore, on notera que l'absence de soudure dans la zone active du support empêche la détérioration de la couche de placage rendant la gaine biocompatible.

Dans l'exemple qui vient d'être décrit, la longueur totale du support est de l'ordre de 200 mm. Bien entendu, cette longueur peut varier en fonction de l'utilisation que l'on désire et plus précisément en fonction de la distance séparant l'endroit du corps où il est introduit de la zone du système cardiovasculaire à traiter.

En ce qui concerne la longueur de la partie distale effilée du fil sur laquelle est enfilée la gaine, elle est de préférence sensiblement inférieure ou égale à 80 mm et cette longueur variera en fonction de la rigidité désirée dans la partie distale du fil.

En se référant maintenant à la figure 2 dans laquelle les éléments identiques à ceux décrits en liaison avec la figure 1 sont désignés par les même références numériques, on voit une variante de réalisation d'un support de guidage selon l'invention.

Selon cette variante, la partie distale 10 du fil 2 comporte, dans sa partie extrême, un tronçon de renfort 28 présentant une section sensiblement égale à la section proximale du fil 2.

Dans ce mode de réalisation, la longueur du tronçon de renfort 28 est de l'ordre de 10 mm. Un tel tronçon de renfort permet à la partie extrême de la gaine d'être supportée et d'empêcher toute déformation sensible dans cette zone de la gaine fortement sollicitée pendant l'introduction du support de sorte que son introduction dans les veines en est facilitée. On comprendra également que la longueur de ce tronçon de renfort ne peut excéder une longueur limite faute de quoi la souplesse de la partie extrême du support diminue et empêche un guidage précis du support dans le système cardiovasculaire et notamment dans le cas où l'extrémité du support doit suivre un parcours très sinueux.

La structure du support selon l'invention permet d'éliminer toute soudure de la partie active du fil 2 de sorte que le risque d'un recuit de la matière formant le fil est supprimé. Qui plus est, outre le fait que l'on supprime une soudure, ce qui rend la fabrication plus rapide et économique, les deux soudures 14 et 24 sont des soudures réalisées de préférence par le procédé de microplasma sans aucun métal d'apport. Dans ce cas, on notera de façon avantageuse que la matière des deux éléments reliés est amenée en fusion de sorte qu'il n'apparaît pas de problème de recuit de matière.

On notera également que, grâce à cette structure du support, on diminue la longueur de la partie effilée, puisqu'il n'est pas nécessaire de prévoir une portion de la partie effilée pour le soudage de la gaine sur le fil. Cela rend par conséquent cette partie plus facile à fabriquer par meulage.

## Revendications

1. Support de guidage pour cathéter comprenant un fil (2) ayant une partie proximale (8) de section sensiblement constante et une partie distale (10) effilée, et une gaine (4) de protection souple s'étendant au moins sur la longueur de la partie distale du fil, le fil (2) étant rendu solidaire de la gaine (4) par l'extrémité libre (16) de sa partie distale (10), le support comprenant en outre un tube (6) s'étendant sur toute la partie (8) du fil (2) non recouverte par la gaine et étant caractérisé en ce que le fil (2) est rendu solidaire de la gaine (4) uniquement par l'extémité libre (16) de sa partie distale (10) au moyen d'une première soudure (14) formant un bout arrondi, une extrémité (17) du tube (6) venant simplement en butée contre la partie proximale (18) de la gaine (4) tandis que l'extrémité opposée (20) du tube est fixée à la partie proximale (8) du fil au moyen d'une seconde soudure (24).

2. Support de guidage selon la revendication 1, caractérisé en ce que le fil (2) comprend en outre à l'extrémité de sa partie distale (10) un tronçon de renfort (28) présentant une section sensiblement égale à la section proximale (8).

3. Support de guidage selon la revendication 1 ou 2, caractérisé en ce que les première (14) et seconde (24) soudures sont des soudures microplasma sans métal d'apport.

4. Support de guidage selon l'une des revendications précédentes, caractérisé en ce que la gaine (4) est formée par un ressort hélicoïdal à spires jointives.

5. Support de guidage selon l'une des revendications précédentes, caractérisé en ce que le tube (6) et la gaine (4) présentent sensiblement le même diamètre.

6. Support de guidage selon la revendication 5, caractérisé en ce que le tube (6) et la gaine (4) présentent un diamètre (D₁, D₂) sensiblement égal ou inférieur à 0,8 mm.

7. Support de guidage selon l'une quelconque des revendications précédentes, caractérisé en ce que la longueur de la partie distale (10) du fil (2) est sensiblement supérieure ou égale à 80 mm.

8. Support de guidage selon l'une quelconque des revendications précédentes, caractérisé en ce que le diamètre (D₃) du bout arrondi est sensiblement égal au diamètre (D₁, D₂) du tube et de la gaine.

9. Support de guidage selon l'une quelconque des revendications précédentes, caractérisé en ce qu'il est revêtu d'une couche (26) d'un matériau à faible coefficient de frottement.

10. Support de guidage selon l'une quelconque des revendications précédentes, caractérisé en ce que le tube (6) et le fil (2) sont réalisés en acier inoxydable.

11. Support de guidage selon l'une quelconque des revendications précédentes, caractérisé en ce que la gaine (4) est réalisée en un matériau radioabile.

12. Support de guidage selon la revendication 11, caractérisé en ce que la gaine (4) est réalisée en un alliage de platine et d'iridium.

## Claims

1. Catheter guide support comprising a wire (2) having a proximal region (8) of substantially constant cross-section and a tapered distal region (10), and a flexible protecting sheath (4) extending at least along the length of the distal region of the wire, the wire (2) being fixed to the sheath (4) at the free end (16) of its distal region (10), the support also comprising a tube (6) extending over the whole region (8) of the wire (2) not covered by the sheath and being characterized in that the wire (2) is fixed to the sheath (4) only at the free end of its distal region (10) of its distal region (10) by means of a first weld (14) forming a rounded tip, one end (17) of the tube (6) simply abutting against the proximal region (18) of the sheath whereas the opposite end (20) of the tube is fixed to the proximal region (8) of the wire by means of a second weld (24).

2. Guide support according to claim 1, characterized in that the wire (2) comprises at the end of its distal region (10) a reinforcing section (28) having a cross-section substantially equal to that of the proximal region (8).

3. Guide support according to claim 1 or 2, characterized in that the first (14) and second (24) welds are microplasma welds without added metal.

4. Guide support according to one of the preceding claims, characterized in that the sheath (4) is formed by a helical spring with adjoining coils.

5. Guide support according to one of the preceding claims, characterized in that the tube (6) and the sheath (4) have substantially the same diameter.

6. Guide support according to claim 5, characterized in that the tube (6) and the sheath (4) have a diameter (D₁, D₂) substantially equal to or less than 0.8 mm.

7. Guide support according to any one of the preceding claims, characterized in that the length of the distal region (10) of the wire (2) is substantially greater or equal to 80 mm.

8. Guide support according to any one of the preceding claims, characterized in that the diameter (D₃) of the rounded tip is substantially equal to the diameter (D₁, D₂) of the tube and the sheath.

9. Guide support according to any one of the preceding claims, characterized in that it is covered by a layer (26) of a material having a low coefficient of friction.

10. Guide support according to any one of the preceding claims, characterized in that the tube (6) and the wire (2) are made of stainless steel.

11. Guide support according to any one of the preceding claims, characterized in that the sheath (4) is made of a radiolabile material.

12. Guide support according to claim 11, characterized in that the sheath (4) is made of an alloy of platinum and iridium.

## Patentansprüche

1. Führungssupport für Katheter, umfassend einen Draht (2) mit einer proximalen Partie (8) im wesentlichen konstanten Querschnitts und einer sich zuspitzenden distalen Partie (10), sowie eine biegsame Schutzhülle (4), die sich über mindestens die Länge der distalen Partie des Drahtes erstreckt, welcher Draht (2) mit der Hülle (4) am freien Ende (16) seiner distalen Partie (10) verbunden ist, wobei der Support ferner ein Rohr (6) umfaßt, das sich auf der gesamten Partie (8) des Drahtes (2), die nicht von der Hülle abgedeckt ist, erstreckt, dadurch gekennzeichnet, daß der Draht (2) mit der Hülle (4) ausschließlich am freien Ende (16) seiner distalen Partie (10) mittels eines ersten, eine abgerundete Spitze bildenden Schweißschmelzpunktes (14) verbunden ist, wobei ein Ende (17) des Rohres (6) einfach an der proximalen Partie (18) der Hülle (4) anliegt, während das entgegengesetzte Ende (20) des Rohres an der proximalen Partie (8) des Drahtes mittels eines zweiten Schweißschmelzpunktes (24) befestigt ist.

2. Führungssupport nach Anspruch 1, dadurch gekennzeichnet, daß der Draht (2) ferner am Ende seiner distalen Partie (10) einen Verstärkungsstumpf (28) aufweist, der einen Querschnitt im wesentlichen gleich dem des proximalen Abschnitts (8) aufweist.

3. Führungssupport nach Anspruch 1 oder 2, dadurch gekennzeichnet, daß der erste (14) und zweite (24) Schweißschmelzpunkt Mikroplasma-Schweißschmelzpunkte ohne Metallzufuhr sind.

4. Führungssupport nach einem der vorangehenden Ansprüche, dadurch gekennzeichnet, daß die Hülle (4) von einer Schraubenfeder mit aneinanderliegenden Windungen gebildet ist.

5. Führungssupport nach einem der vorangehenden Ansprüche, dadurch gekennzeichnet, daß das Rohr (6) und die Hülle (4) im wesentlichen gleichen Durchmesser aufweisen.

6. Führungssupport nach Anspruch 5, dadurch gekennzeichnet, daß das Rohr (6) und die Hülle (4) einen Durchmesser (D₁, D₂) im wesentlichen gleich oder kleiner 0,8 mm aufweisen.

7. Führungssupport nach einem der vorangehenden Ansprüche, dadurch gekennzeichnet, daß die Länge der distalen Partie (10) des Drahtes (2) wesentlich größer oder gleich 80 mm ist.

8. Führungssupport nach einem der vorangehenden Ansprüche, dadurch gekennzeichnet, daß der Durchmesser (D₃) der abgerundeten Spitze im wesentlichen gleich dem Durchmesser (D₁, D₂) des Rohres und der Hülle ist.

9. Führungssupport nach einem der vorangehenden Ansprüche, dadurch gekennzeichnet, daß er mit einer Beschichtung (26) aus einem Material geringen Reibungskoeffizienten bedeckt ist.

10. Führungssupport nach einem der vorangehenden Ansprüche, dadurch gekennzeichnet, daß das Rohr (6) und der Draht (2) aus korrosionsfestem Stahl hergestellt sind.

11. Führungssupport nach einem der vorangehenden Ansprüche, dadurch gekennzeichnet, daß die Hülle (4) aus einem radiologisch abbildbaren Material hergestellt ist.

12. Führungssupport nach Anspruch 11, dadurch gekennzeichnet, daß die Hülle (4) aus einer Platin-Iridium-Legierung hergestellt ist.
